## Europäisches Patentamt

## European Patent Office

## Office européen des brevets

(19)

(11) Publication number: **0 134 436**
**B1**

(12)

# EUROPEAN PATENT SPECIFICATION

(45) Date of publication of patent specification: **07.01.88**

(51) Int. Cl.⁴: **A 61 M 1/14**

(21) Application number: **84106973.5**

(22) Date of filing: **18.06.84**

(54) System for extracorporeal blood treatment.

(30) Priority: **30.06.83 SE 8303742**

(43) Date of publication of application:
**20.03.85 Bulletin 85/12**

(45) Publication of the grant of the patent:
**07.01.88 Bulletin 88/01**

(84) Designated Contracting States:
**BE CH DE FR GB IT LI LU NL**

(56) References cited:
**CH-A- 594 418**
**CH-A- 623 134**
**GB-A-1 601 855**
**GB-A-2 110 564**

(73) Proprietor: **Gambro Lundia AB**
**Box 10101**
**S-220 10 Lund (SE)**

(72) Inventor: **Flank, Hans Peder**
**Kommendörsgatan 7C**
**S-211 50 Malmö (SE)**
Inventor: **Staehr, Peter Robert**
**Hästad 27:1**
**S-225 90 Lund (SE)**

(74) Representative: **Boberg, Nils Gunnar Erik**
**Gambro AB Patent Department Box 10101**
**S-220 10 Lund (SE)**

Note: Within nine months from the publication of the mention of the grant of the European patent. any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa. England.

## Description

Technical Field

The present invention relates to a system for extracorporeal blood treatment comprising a monitor for the control of the course of the treatment, a treatment unit for the treatment itself and a tube system for the conducting of blood from a patient to the treatment unit and back to the patient under control by the monitor, wherein said tube system is in the form of a substantially rigid cassette which is adapted so as to be fixed on, and connected to, the monitor in such a manner that the desired functions such as the control of pressure and/or temperature and/or pumping of the blood are transmitted directly between the monitor and the cassette through coupling of the latter to the monitor and via two flexible tubes to the patient and via two further connecting ducts to the treatment unit itself.

The invention is intended in particular to be applied in connection with haemodialysis, haemofiltration and immunotherapy. It will be clear, however, to those versed in the art that it can be applied advantageously in connection with any kind of extracorporeal blood treatment.

Background Art

In extracorporeal blood treatment normally different types of tube systems are used which conduct the blood to and from the patient, to and from the treatment unit, e.g. a dialyzer, and to and from different functional positions, such as stations for pumping, pressure measurement, sampling, injection etc. After fixing the tube system to a control monitor and connecting it to the same, the system is connected to the patient, dialyzer or some other treatment unit. The more functions are to be included, the more complicated becomes the tube system and its fixing and connecting up respectively.

Attempts have also been made to integrate certain functions in cassettelike units to be fixed to the dialyzer and/or the front of the control monitor. See for example GB patents 1 601 855, 1 601 856 and GB application 2 110 564, US patents 4 211 597 and 4 231 871 and European patent application 82.420073, published under No. EP 0 069 029.

Disclosure of Invention

In cassette systems of the abovementioned kind the monitor and the cassette must of course be adapted accurately to one another. To make it possible nevertheless to use the monitor for conventional tube systems a special cassettelike holder has been provided in accordance with the invention, by means of which the control function of the monitor is arranged to be transmitted to the conventional tube system when it is fixed onto the monitor by means of the holder at the place intended for fixing the cassette. Owing to this possibility of using also conventional tube systems all the advantages of the cassette can be obtained whilst retaining the freedom of producing, for example in small series, special tube systems for special treatments.

The monitor preferably comprises one or more pressure transducers which are adapted so that they are acted upong mechanically by the cassette when the same is fixed to the monitor. For example the cassette may be adapted so that it presses against the pressure transducer with the help of a flexible wall which by means of suction is fastened to the pressure transducer for the measurement of the absolute positive or negative pressure in the cassette. In this way the pressure can be measured without any direct contact between the blood and the pressure transducer. Neither are any duct connections comprising liquid-tight filters or membranes required as customary otherwise in connection with blood pressure measurement in extracorporeal systems.

The monitor appropriately comprises a source of positive and/or negative pressure arranged so that on fixing of the cassette it is made to communicate directly with the air space in a drip chamber arranged in the cassette so as to regulate the level therein. In this way good safety against air embolism is achieved, especially by inclusion of the drip chamber in the cassette. The connecting up of the level regulator thus takes place automatically and cannot be forgotten.

The monitor moreover may comprise in conventional manner one or more driving elements for one or more tube pumps. The tube segment(s) entering into these pumps preferably consist of segments firmly joined to the cassette. In this manner a correct connecting up of the pump function is facilitated. It can thus be ensured that segments are not connected up backwards which is possible in many conventional tube systems.

The cassette may further comprise one or more expansion chambers. These are required, for example, when the system in accordance with the invention is used for so-called "single-needle" technique and the actual treatment unit cannot accommodate the varying blood volumes which occur if in accordance with this technique blood is alternately withdrawn from, and returned to, a patient with the help of only one cannula.

The cassettelike holder appropriately comprises outlets hermetically attachable to the pressure transducer of the monitor which are provided with means for connection to appropriate pressure measuring positions in the conventional tube system. Through this the coupling of this system to the monitor is of course facilitated.

The cassettelike holder may comprise moreover an outlet hermetically connectable to the said source of positive and/or negative pressure which is provided with means for connection to a drip chamber forming part of the conventional tube system for the regulation of the level in this drip chamber which should be capable of being readily fixed to the holder.

Furthermore, the cassettelike holder and/or the monitor should comprise fastening devices for the fixing of one or more pump tube segments forming part of the conventional tube system in a firm position or positions in relation to one or more tube pump driving elements arranged on the monitor.

Brief Description of Drawings

Fig. 1 shows a cassette comprising a pump segment in a position before a monitor front ready to be fixed to this front.

Fig. 2 shows in the same manner a cassette with two pump segments.

Fig. 3 and 4 show schematic and slightly modified flow diagrams for the cassette according to Fig. 1 and 2.

Fig. 5 and 6 show in corresponding manner a flow diagram when the cassette according to Fig. 3 has been replaced by a holder and a conventional tube set.

Fig. 7—11 are two views perpendicular to each other and three sections respectively through a slightly modified holder.

Fig. 12—14 show three views perpendicular to each other of a cassette adapted for blow forming and with a shape adapted to fit essentially the same monitor as the holder according to Fig. 7—11.

Fig. 15 finally shows an example of how the pressure transmission between the cassette and the monitor can occur.

Best Mode of Carrying Out the Invention
*Single Pump System*

The invention is particularly well suited for application dialysis. For this reason and for the sake of simplicity the embodiments included in the following are described with reference to dialysis although they can of course also be used in other extracorporeal blood treatment.

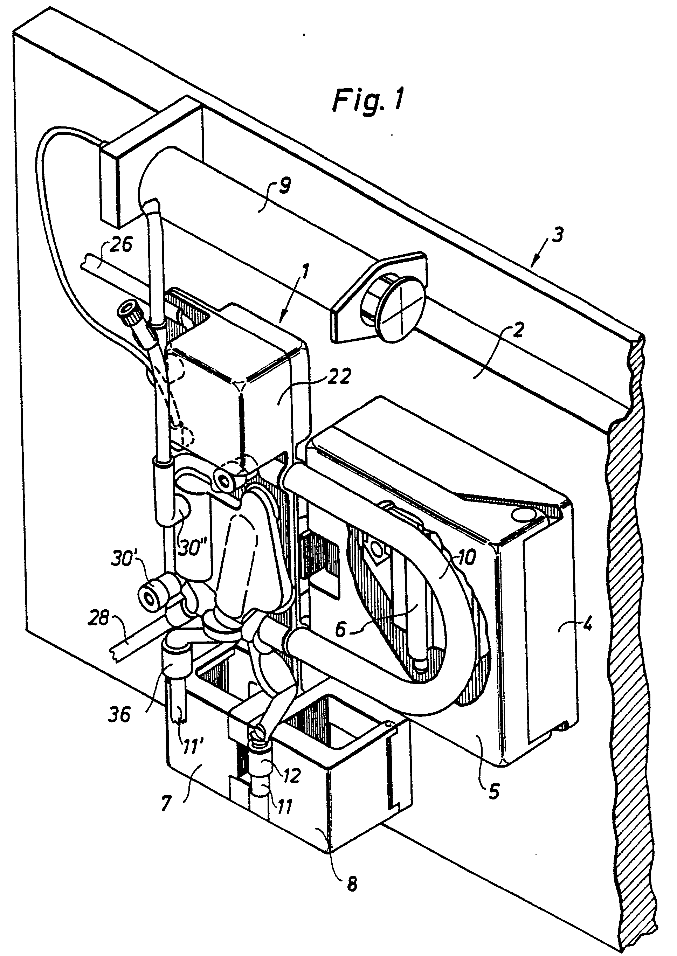

In Fig. 1 thus a cassette 1 is shown ready to be fixed to the front 2 of a dialysis monitor 3. On the outside of the front a tube pump 4 is mounted. Inside the outer door 5 of the pump is indicated the pump rotor 6. The front carries moreover on its outside conventional artery and vein clamps 7 and 8 and a heparin pump 9. At the back of the cassette are concealed various further connecting elements for the transmission of functions between the cassette and the monitor. This transmission thus can take place in both directions.

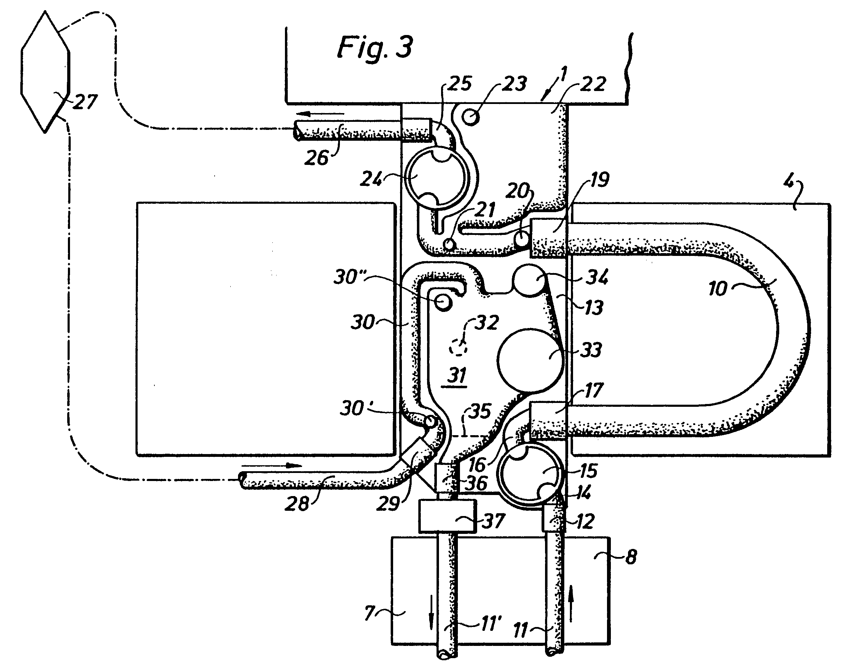

When the cassette 1 is to be fixed the door 5 is opened so that the pump segment 10 can be placed around the pump rotor 6. Then the door 5 is shut and can thus serve as the sole fixing element for the cassette 1. It is appropriate, however, to provide further fixing either with the help of mechanical means or in that the whole cassette 1 is fastened to the front 2 by suction. Other components included in the cassette 1 will be referred to later, in particular in connection with the description of Fig. 3 and 4 and 12—14.

*Double Pump System*

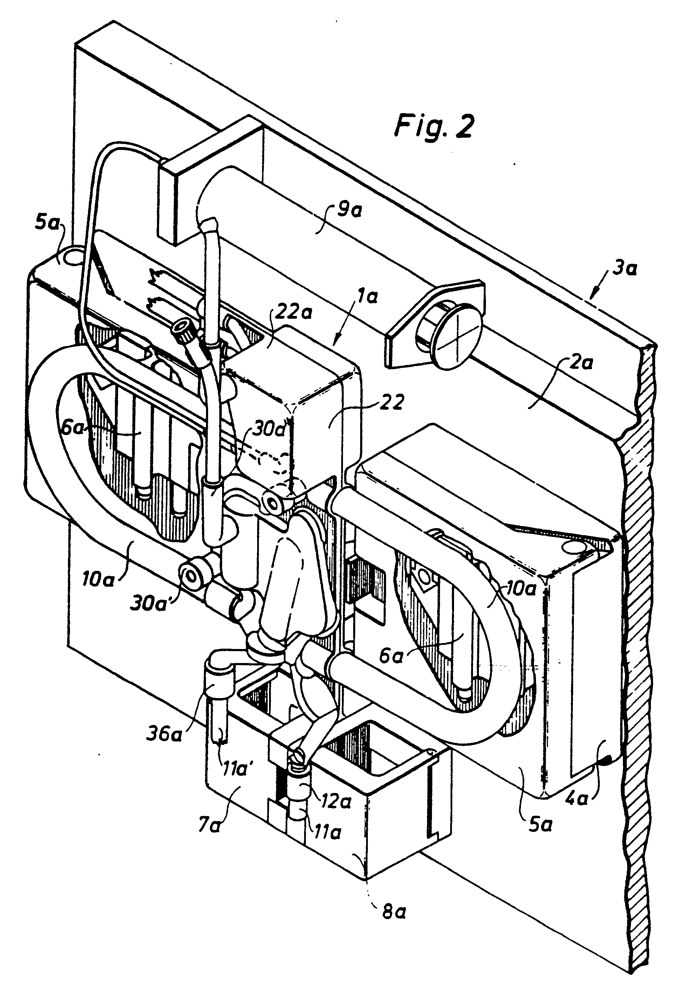

In Fig. 2 is shown an alternative embodiment where the cassette is provided with two pump segments. However, as the construction in principle is the same, identical reference designations have been used but with the added letter *a*. Thus in Fig. 2 among others the following components are present:

1a — Cassette with two pump segments
2a — Monitor front
3a — Monitor
4a — Pump
5a — Pump door
6a — Pump rotor
7a — Vein clamp
8a — Artery clamp
9a — Heparin pump
10a and 10b — Pump segment

Flow Diagram for Single Pump System

Fig. 3 shows schematically and slightly modified the flow diagram when the cassette in accordance with Fig. 1 is used. The blood is conducted from the patient via a tube 11 through the artery clamp 8 to the inlet 12 of the central part 13 of the cassette 1. From the inlet 12 the blood is passed via a short duct 14 into a first pressure measuring head 15 which may be of substantially the same construction as that which forms the subject of EP—A—0 130 441 submitted at the same time. The blood is then conducted via the duct 16 to the pump inlet 17 from which a pump segment 10 leads to a pump outlet 19. Numeral 20 designates a so-called injection port which in the present case is intended for sampling of non-purified blood. Such sampling is carried out by inserting a cannula through a self-closing material, e.g. silicone rubber. Numeral 21 designates a connecting nozzle for a heparin tube (not shown). This connecting nozzle sits straight before the inlet to an expansion chamber 22. The level in the expansion chamber 22 can be regulated by air being sucked out or fed in through a nipple 23. After the blood has passed the expansion chamber 22 it arrives at a second pressure measuring head 24 which may be of the same type as the pressure measuring head 15. Thereafter the blood is conducted via the duct 25 out into the tube 26 which passes it to the dialyzer 27, indicated symbolically. From this dialyzer the blood is returned to the central part 13 via the tube 28 and an inlet 29. Just before this inlet there is a further injection port 30′ in the duct 30 through which sampling of purified blood can be carried out. The duct 30 opens into a drip chamber 31 with an infusion position 30″, a float switch 32 and a pressure measuring head 33 which again may be of substantially the same type as the pressure measuring head 15. The float switch 32 is adapted to control a pump arranged in the monitor which is connected to a connecting nozzle 34 through which air can be pumped into, or sucked out from, the drip chamber so as to regulate the level. In the lower part of the drip chamber a strainer 35 is indicated symbolically just before the outlet 36. From this outlet 36 the blood first passes and air switch 37 and then the vein clamp 7 before being returned again to the patient. The level regulating connection 23 and 34 may consist of flexible tubes connected to the nozzles intended for connection to suitable pumps or other sources of pressure. Alternatively the cassette may be adapted so that these points are tightly pressed directly against suction and/or outlets on the monitor front.

## Flow Diagram for the Double Pump System

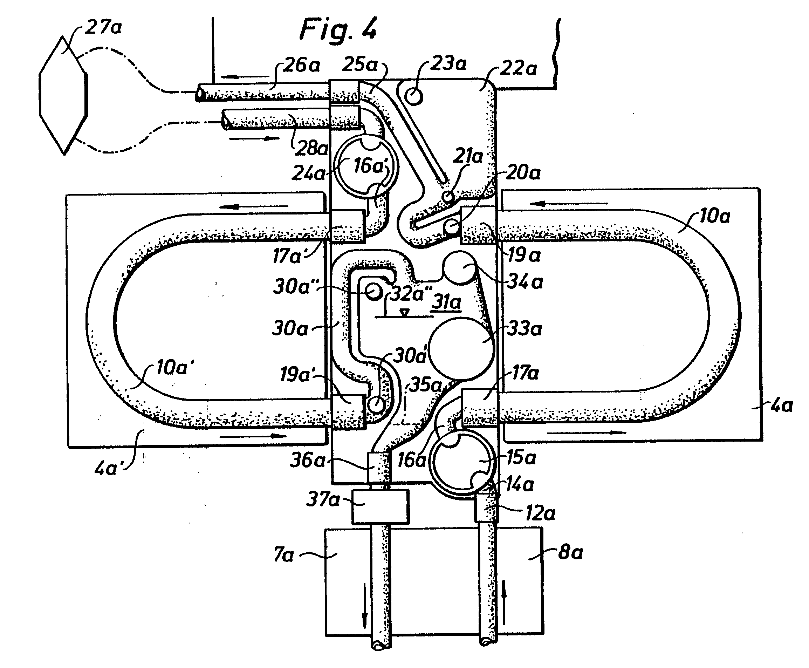

In Fig. 4, in the same manner as in Fig. 3, the flow diagram for a cassette is shown which in this case is· provided with two pump segments. The construction in principle is the same so that identical reference designations have been used, but with the added letter *a*.

The blood is thus conducted via the artery clamp 8a, the inlet 12a, the duct 14a, the pressure measuring head 15a and the duct 16a to the inlet 17a of the pump 4a. The blood is then conducted via the pump segment 10a, the outlet 19a and the duct 25a past the injection port 20a and the slightly modified expansion chamber 22a with heparin supply connection 21a and level regulating connection 23a. Without passing any pressure measurement the blood is then conducted with the help of the tube 26a to the dialyzer 27a shown schematically. From there the blood is then conducted through the tube 28a to a pressure gauge 24a which has the same position here as the pressure gauge 24, but which measures the outgoing pressure from the dialyzer instead of the incoming one. Via a duct 16a', an inlet 17a', a pump segment 10a' and an outlet 19a' the blood is then pumped via a duct 30a with an injection port 30a' to a drip chamber 31a constructed essentially in the same manner as the drip chamber 31, that is to say with an infusion position 30a'', a float switch 32a'', a pressure measuring head 33a, a connection for level regulation 34a and a strainer 35a. Finally the blood is returned again to the patient via the outlet 36a, the air switch 37a and the vein clamp 7a.

## Holder for Conventional Tube System

To allow the monitor forming part of the system in accordance with the invention to be used also for conventional tube systems, a special holder for such systems has been provided in accordance with the invention.

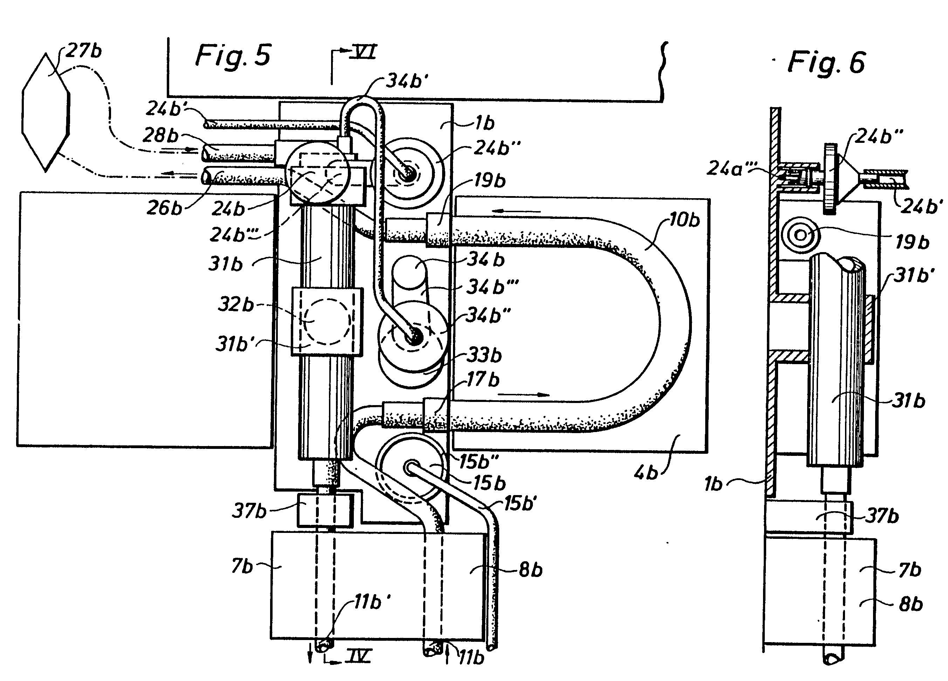

A front view of this holder is shown in Fig. 5. Fig. 6 shows moreover a section along the line VI—VI in Fig. 5.

As the function of certain components in Fig. 5 and 6 in principle agrees with those of corresponding components in Fig. 3 identical reference designations have been used as far as possible, but with the added letter *b*. The blood is thus fed to the conventional tube system through the duct 11b via the artery clamp 8b. With the help of the pump 4b with its inlet 17b, outlet 19b and pump segment 10b the blood is then pumped via a duct 26b to the dialyzer 27b shown schematically. The blood is then returned via the duct 28b to the holder 1b and more particularly to a drip chamber

31b fixed to this holder. The drip chamber is fixed to the holder 1b with the help of a fixing device 31b' which comprises within it a float switch 32b. This switch, in the same manner as the switches 32 and 32a, is adapted to control a pump arranged in the monitor which via the level regulating outlet 34b, a duct 34b''' and a duct 34b' controls the level in the drip chamber by pumping of air to and from this chamber. The duct 34' is used also for transmitting the pressure from the drip chamber 31b to the pressure measuring head 33b for measuring the venous pressure. In the same manner the arterial pressure is measured with the help of a special pressure measuring head 15b via a duct 15b' which is connected to the duct 11 at a point (not shown) before the pump 4b. In the same manner the pressure measuring head 24b is used for measuring the pressure via a duct 24b' at a point (not shown) before or after the dialyzer 27b. The tubes 15b', 34b' and 24b' are all connected to respective pressure measuring heads via conventional so-called disc filters 15b'', 34b'' and 24b'' respectively. In the lastnamed case this takes place via a duct 24b'''.

From the drip chamber 31b the blood is passed through a tube 11b' via an air switch 37b and the vein clamp 7b back to the patient.

## Alternative Technical Construction of Holder

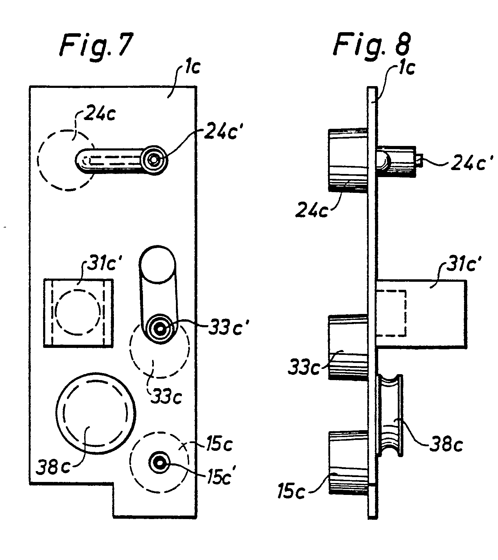

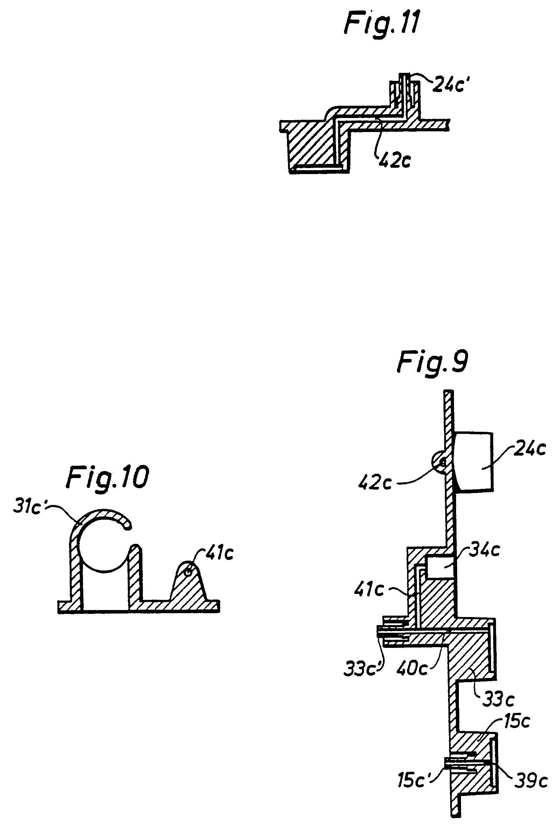

Fig. 7—11 show a somewhat modified construction of the holder in accordance with Fig. 5 and 6. For reasons of simplicity and for the sake of clarity in this case without any conventional tube system connected. However, as the construction in principle is the same, identical reference designations have been used, but with the added letter *c*. Three pressure measuring heads are thus designated 15c, 24c and 33c and their tube connection positions are designated 15c', 24c' and 33c'. A fixing arrangement 31c' corresponds to the fixing arrangement 31b' for the drip chamber 31b. To prevent the formation of kinks on tubes belonging to the conventional tube system, the holder 1c is provided with a guide wheel 38c. Numerals 39c, 40c, 41c and 42c finally refer to different ducts which connect the tube connecting positions 15c', 33c' and 24c' respectively to the particular pressure transducer in the monitor and to a level regulation outlet 34c corresponding to the outlets 34, 34a and 34b.

## Best Mode of Carrying Out the Cassette

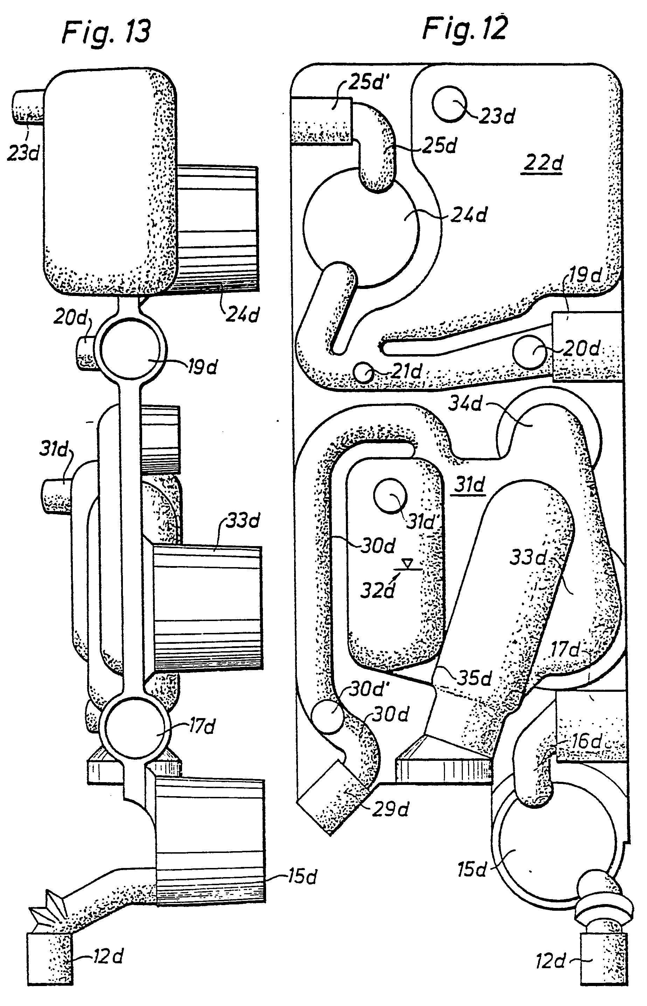

In Fig. 12—14 is shown a preferred embodiment of the cassette adapted for one pump segment and for manufacture by blow forming. By means of this manufacturing process a relatively inexpensive cassette suitable for throw-away use can be produced. Alternatively, however, the cassette can also be made, for example, by injection moulding.

The blow-formed version corresponds in principle with that according to Fig. 1 and 3. Hence identical reference designations have been used with the added letter *d*. The blood is thus fed via the inlet 12d, the pressure measuring head 15d, the duct 16d and the pump inlet 17d to the pump (not shown). From the outlet 19d of the latter the blood is conducted past the injection port 20d and the heparin inlet 21d at the inlet to the expansion chamber 22d with its level regulating nipple 23d. Subsequently the blood is conducted via the pressure measuring head 24d and the duct 25d to the attachment 25d' for a tube (not shown) intended to be connected to the dialyzer (also not shown).

From the dialyzer the blood is returned to a corresponding attachment 29d for another tube corresponding, for example, to the tube 28 in Fig. 3. Via a line or duct 30d the blood is then led past a sampling position 30d' for the sampling of purified blood. The drip chamber 31d has been given a slightly modified shape so as to include a cylindrical strainer 35d. In the drip chamber are present once more an infusion position 31d', a float switch 32d, a pressure measuring head 33d and a connection position 34d intended for level regulation. The function is thus the same as for the cassette described earlier. A more detailed description of the function is therefore not required.

Pressure Transmission

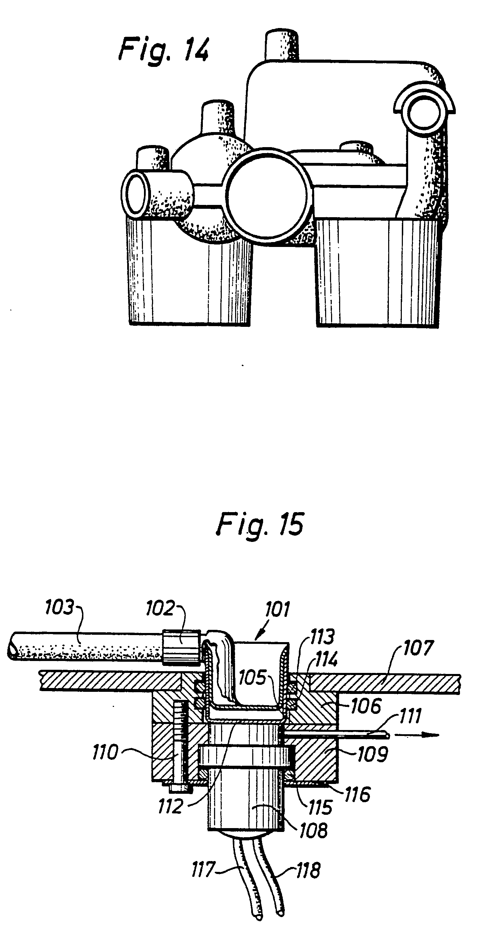

To illustrate the function of the abovementioned pressure measuring head reference is made to Fig. 15 which shows separately such a pressure measuring head 101 sunk into a machine front 107 corresponding to the front 2 of the monitor 3 shown in Fig. 1. Thus in Fig. 15 is shown a container 101 with an inlet 102 which via a tube 103 communicates with the medium whose pressure is to be measured. If a through-flowing medium is measured, the container 101 is of course also provided with an outlet.

In operating condition the container 101 is sunk into a cavity 105 in a machine component 106 which is fixed into a front plate 107, e.g. the front of a monitor for the control of haemodialysis or haemofiltration. On the machine component 106 a pressure-sensitive element 108 is fastened with the help of a further machine component 109. This component may be screwed on, for example, by means of a bolt 110. The pressure-sensitive element 108 may be constituted, for example, of a piezoelectric pressure pick-up. Alternatively it may comprise a wire strain pick-up or any other suitable pressure measuring element. With the help of a duct 111 the cavity 105 can be made to communicate with a source of vacuum (not shown). In that manner a pressure-transmitting wall part 112 of the container 101 is fastened by suction onto the pressure-sensitive element 108. The pressure-transmitting wall part 112 and the pressure-monitoring surface of the pressure-sensitive element 108 are sealed against the surrounding atmosphere with the help of annular seals 113, 114 and 115. The lastmentioned seal 115 is thus situated inside a lockwasher 116. Finally an electric connection to the pressure-sensitive element is marked 117 and 118 in Fig. 15.

If a conventional tube system is connected instead with the help of pressure-measuring heads of the type as shown in Fig. 5—11, it is only necessary for the vacuum duct 111 to be closed before the pressure is measured.

If further information regarding the construction and function of the pressure-measuring heads is required, reference is made to EP—A—0 130 441 submitted at the same time, from which Fig. 15 has been taken.

The invention is not limited only to the embodiments described above, but can be varied within the scope of the following claims. In particular, for example, if it is desired to change from the blow forming to the injection moulding process, the shape of the components can be modified in substantial respects. In practice it has been found that by blow forming relatively thin bases for the pressure measuring heads can be obtained directly in the actual blowing process. If injection moulding is used instead it may become necessary to manufacture the pressure measuring heads with open bases covered by welded-on or glued-on thin membranes.

## Claims

1. A system for extracorporeal blood treatment comprising a monitor (3) for the control of the course of the treatment, a treatment unit (27) for the treatment itself and a tube system for the conducting of blood from a patient to the treatment unit (27) and back to the patient under control by the monitor (3), wherein said tube system is in the form of a substantially rigid cassette (1) which is adapted so as to be fixed on, and connected to, the monitor (3) in such a manner that the desired functions such as the control of pressure and/or temperature and/or pumping of the blood are transmitted directly between the monitor and the cassette through coupling of the latter to the monitor and via two flexible tubes (11, 11') to the patient and via two further connecting ducts (26, 28) to the treatment unit (27) itself, characterized by a cassettelike holder (1b) for a conventional tube system (11b, 15b, 10b, 26b, 28b, 31b) by means of which the control function of the monitor (3) is arranged to be transmitted to the conventional tube system when it is fixed to the monitor (3) by means of the holder (1b) at the place intended for fixing the cassette (1).

2. A system in accordance with claim 1, characterized in that the monitor (3) comprises one or more pressure transducers (108) which are adapted so that they are acted upon mechanically by the cassette (1) when the same is fixed to the monitor.

3. A system in accordance with claim 2, characterized in that the cassette (1) is adapted so that it presses against the pressure transducer (108) with the help of a flexible wall (112) which by means of suction is fastened to the pressure transducer (108) for the measurement of the absolute positive or negative pressure in the cassette (1).

4. A system in accordance with claim 1, characterized in that the monitor (3) comprises a source of positive and/or negative pressure, arranged so that on fixing of the cassette (1) it is made to communicate directly with the air space in a drip chamber (3) arranged in the cassette (1) so as to regulate the level therein.

5. A system in accordance with claim 1, characterized in that the monitor (3) comprises one or more driving elements (6) for one or more tube pumps (4), the tube segment(s) (10) entering into these pumps (4) being constituted of segments (10) firmly joined to the cassette.

6. A system in accordance with claim 1, characterized in that the cassette (1) comprises one or more expansion chambers (22).

7. A system in accordance with claims 1 and 2, characterized in that the cassettelike holder (1b) comprises outlets (15b, 24b, 33b) hermetically attachable to the pressure transducer (108) of the monitor which are provided with means (15b', 15b'', 24b', 24b'', 34b', 34b'') for connection to appropriate pressure measuring positions in the conventional tube system.

8. A system in accordance with claims 1 and 4, characterized in that the cassettelike holder (1b) comprises an outlet (34b) hermetically connectable to the said source of positive and/or negative pressure which is provided with means (34b', 34b'', 34b''') for connection to a drip chamber (31b) forming part of the conventional tube system for the regulation of the level in the said drip chamber.

9. A system in accordance with claims 1 and 5, characterized in that the cassettelike holder and/or the monitor comprise fastening devices for the fixing of one or more pump segments forming part of the conventional tube system in a firm position or positions in relation to one or more tube pump driving elements arranged on the monitor.

**Patentansprüche**

1. System bzw. Aufbau für die Behandlung von Blut außerhalb des Körpers mit einer Steuereinheit bzw. Monitor (3) für die Steuerung des Behandlungsablaufes, einer Behandlungseinheit (27) für die Behandlung selbst und einem Rohr- bzw. Schlauchsystem für die Leitung von Blut von einem Patienten zu der Behandlungseinheit (27) und zurück zu dem Patienten unter Steuerung durch den Monitor (3), wobei das Schlauchsystem die Form einer im wesentlichen festen Kassette (1) hat, welche so ausgelegt ist, daß sie in der Weise an dem Monitor (3) befestigt und angeschlossen werden kann, daß die gewünschten Funktionen, wie die Steuerung des Druckes und/oder der Temperatur und/oder des Pumpens von Blut, direkt zwischen dem Monitor und der Kassette übertragen werden durch Ankoppeln der letzteren an den Monitor und über zwei flexible Schläuche (11, 11') zu dem Patienten und über zwei weitere Verbindungsleitungen (26, 28) zu der Behandlungseinheit (27) selbst, gekennzeichnet durch einen kassettenartigen Halter (1b) für ein konventionelles Schlauchsystem (11b, 15b, 10b, 26b, 28b, 31b), mit Hilfe dessen erreicht wird, daß die Steuerfunktion des Monitors (3) auf das konventionelle Schlauchsystem übertragen wird, wenn es an dem Monitor (3) mit Hilfe des Halters (1b) an der für das Befestigen der Kassette (1) vorgesehenen Stelle befestigt wird.

2. System nach Anspruch 1, dadurch gekennzeichnet, daß der Monitor (3) einen oder mehrere Druckübertrager (108) aufweist, welche so ausgelegt sind, daß die Kassette (1) mechanisch auf die wirkt, wenn sie an dem Monitor befestigt wird.

3. System nach Anspruch 2, dadurch gekennzeichnet, daß die Kassette (1) so ausgelegt ist, daß sie gegen den Druckübertrager (108) mit Hilfe einer flexiblen Wand (112) drückt, welche mit Hilfe eines Saugdruckes an dem Druckübertrager (108) zum Messen des absoluten positiven oder negativen Druckes in der Kassette (1) befestigt ist.

4. System nach Anspruch 1, dadurch gekennzeichnet, daß der Monitor (3) eine Quelle positiven und/oder negativen Druckes aufweist, welche derart angeordnet ist, daß sie durch das Befestigen der Kassette (1) in direkte Verbindung mit dem Luftraum in einer Tropfkammer (3) gebracht wird, welche in der Kassette (1) angeordnet ist, um das Niveau darin zu regulieren.

5. System nach Anspruch 1, dadurch gekennzeichnet, daß der Monitor (3) eine oder mehrere Antriebselemente (6) für eine oder mehrere Schlauchpumpen (4) aufweist, wobei der (die) Schlauchabschnitt(e) (10), welche in diese Pumpen (4) hineinführen, aus fest mit der Kassette verbundenen Abschnitten (10) bestehen.

6. System nach Anspruch 1, dadurch gekennzeichnet, daß die Kassette (1) eine oder mehrere Dehnungskammern (22) aufweist.

7. System nach Anspruch 1 und 2, dadurch gekennzeichnet, daß der kassettenartige Halter (1b) Auslässe (15b, 24b, 33b) aufweist, welche dicht schließend an dem Druckübertrager (108) des Monitors anbringbar sind und welche mit Mitteln (15b', 15b'', 24b', 24b'', 34b', 34b'') zum Anschluß an geeignete Druckmeßstellen in dem konventionellen Schlauchsystem versehen sind.

8. System gemäß den Ansprüchen 1 und 4, dadurch gekennzeichnet, daß der kassettenartige Halter (1b) einen Auslaß (34b) aufweist, welcher dicht schließend mit der Quelle positiven und/oder negativen Druckes verbindbar ist, welche mit Mitteln (34b', 34b'', 34b''') versehen ist für die Verbindung zu einer Tropfkammer (31b), welche einen Teil des konventionellen Schlauchsystems zur Regulierung des Niveaus in der Tropfkammer bildet.

9. System gemäß Ansprüchen 1 und 5, dadurch gekennzeichnet, daß der kassettenartige Halter und/oder der Monitor Befestigungsvorrichtungen aufweisen für die Befestigung einer oder mehrerer Pumpenabschnitte, welche einen Teil eines konventionellen Schlauchsystems bilden, in einer sicheren Lage oder Positionen in Bezug auf eine oder mehrere Pumpenantriebselemente, welche an dem Monitor angebracht sind.

**Revendications**

1. Système pour le traitement extra-corporel du sang, comprenant un dispositif de pilotage (3) pour la commande du déroulement du traitement, une unité de traitement (27) pour le traitement lui-même et un système tubulaire pour amener le sang d'un patient à l'unité de traitement (27) et le renvoyer au patient sous la commande du dispositif de pilotage (3), ledit système tubulaire étant sous la forme d'une cassette sensiblement rigide (1) qui est conçue pour être fixée sur le dispositif de pilotage (3) et raccordée à celui-ci d'une manière telle que les fonctions désirées, par exemple le contrôle de pression et/ou de température et/ou le pompage du sang, sont transmises directement entre le dispositif de pilotage et la cassette par accouplement de cette dernière au dispositif de pilotage et, par l'intermédiaire de deux tubes souples (11, 11'), au patient et, par l'intermédiaire de deux autres conduits de liaison (26, 28), à l'unité de traitement (27) elle-même, caractérisé en ce qu'il comprend un support en forme de cassette (1b) pour un système tubulaire usuel (11b, 15b, 10b, 26b, 28b, 31b), au moyen duquel les fonctions de commande du dispositif de pilotage (3) peuvent être transmises au système tubulaire usuel lorsque celui-ci est fixé au dispositif de pilotage (3) au moyen du support (1b) à l'endroit prévu pour la fixation de la cassette (1).

2. Système suivant la revendication 1, caractérisé en ce que le dispositif de pilotage (3) comprend un ou plusieurs transducteurs de pression (108) qui sont prévus de sorte que la cassette (1) agit mécaniquement sur eux lorsqu'elle est fixée au dispositif de pilotage.

3. Système suivant la revendication 2, caractérisé en ce que la cassette (1) est conçue de sorte qu'elle presse contre le transducteur de pression (108) par l'intermédiaire d'une paroi souple (112) qui est fixée, par des moyens d'aspiration, au transducteur de pression (108) pour la mesure de la pression absolue positive ou négative dans la cassette (1).

4. Système suivant la revendication 1, caractérisé en ce que le dispositif de pilotage (3) comprend une source de pression positive et/ou négative, agencée de sorte que, lors de la fixation de la cassette (1), elle est mise en communication directement avec l'espace d'air dans une chambre de goutte-à-goutte (3) prévue dans la cassette (1), de manière à réguler le niveau dans cette chambre.

5. Système suivant la revendication 1, caractérisé en ce que le dispositif de pilotage (3) comprend un ou plusieurs éléments d'entraînement (6) pour une ou plusieurs pompes à tube (4), le ou les segments de tube (10) entrant dans ces pompes (4) étant constitués de segments (10) reliés solidairement à la cassette.

6. Système suivant la revendication 1, caractérisé en ce que la cassette (1) comprend une ou plusieurs chambres d'expansion (22).

7. Système suivant les revendications 1 et 2, caractérisé en ce que le support (1b) en forme de cassette comprend des sorties (15b, 24b, 33b) qui peuvent être fixées de façon hermétique au transducteur de pression (108) du dispositif de pilotage et qui comportent des moyens (15b', 15b'', 24b', 24b'', 34b', 34b'') pour raccordement à des positions de mesure de pression appropriées dans le système tubulaire usuel.

8. Système suivant les revendications 1 et 4, caractérisé en ce que le support (1b) en forme de cassette comprend une sortie (34b), connectable hermétiquement à ladite source de pression positive et/ou négative, qui comporte des moyens (34b', 34b'', 34b''') pour le raccordement à une chambre de goutte-à-goutte (31b) faisant partie du système tubulaire usuel, pour la régulation du niveau dans ladite chambre de goutte-à-goutte.

9. Système suivant les revendications 1 et 5, caractérisé en ce que le support en forme de cassette et/ou le dispositif de pilotage comprennent des dispositifs de fixation, pour fixer ou un plusieurs segments de pompe, faisant partie du système tubulaire usuel, dans une ou des positions déterminées par rapport à un ou plusieurs éléments d'entraînement de pompe à tube prévus sur le dispositif de pilotage.

## Fig. 1

Fig. 2

*Fig. 4*

Fig. 6

Fig. 5

## Fig.7

24c
24c'
1c
31c'
33c'
33c
38c
15c
15c'

## Fig. 8

1c
24c'
24c
31c'
33c'
33c
38c
15c

*Fig.11*

24c'

42c

*Fig.10*

31c'

41c

*Fig.9*

24c

42c

34c

41c

33c'  40c

33c

15c

15c'  39c

# Fig. 13

# Fig. 12

# Fig. 14

# Fig. 15